# EUROPEAN PATENT SPECIFICATION

(11) **EP 1 806 108 B1**
(45) Date of publication and mention of the grant of the patent: **05.05.2010**
(21) Application number: 05785715.3
(22) Date of filing: 22.09.2005
(51) Int. Cl.: A61B 18/12, A61B 18/00

(54) **ULTRASONIC OPERATING SYSTEM**
ULTRASCHALLSYSTEM
SYSTEME DE FONCTIONNEMENT ULTRASONIQUE

(30) Priority: 27.09.2004 JP 2004280012
(43) Date of publication of application: 11.07.2007
(73) Proprietor: Olympus Corporation, Tokyo 151-0072 (JP)
(72) Inventor: SHIMIZU, Koh, Hachioji-shi, Tokyo 1920032 (JP)
(74) Representative: von Hellfeld, Axel
(86) International application number: PCT/JP2005/017458
(87) International publication number: WO 2006/035659

(56) References cited:
- WO-A-2004/037104
- JP-A- 11 318 919
- JP-A- 2002 238 919
- JP-A- 2003 033 369
- US-A1- 2001 029 315
- US-A1- 2002 115 917
- US-A1- 2003 040 672
- US-A1- 2005 043 828

## Description

### Technical Field

The present invention relates to an ultrasonic surgery system with a changeover mechanism for output modes of an ultrasonic output and a high-frequency electric current output.

### Background Art

A surgical operator generally performs a surgical operation by changing over output modes, prior to or during a treatment, of a hand piece (also called a Hand Instrument) held by the operator, which is a part of an ultrasonic surgical operation apparatus for use in a surgical operation. Known changeover mechanisms for output modes include disclosed patent documents as noted in the following.

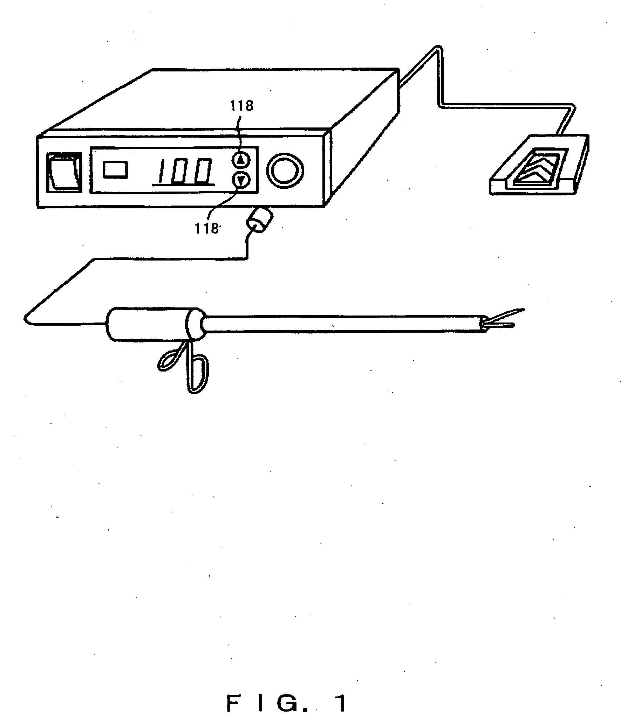

An ultrasonic surgical operation apparatus disclosed in patent document 1 is equipped with a setup switch 118 for presetting an ultrasonic output value and a control circuit for changing over operation states so that an ultrasonic output becomes the set output value after the start of a treatment as shown by Fig. 1. A larger ultrasonic oscillation than the set output value of an ultrasonic wave for a normal operation is output only at start of an ultrasonic treatment. Therefore, the time for obtaining an effect of a treatment, such as incision and coagulation, appearing at the time of an ultrasonic treatment is shortened.

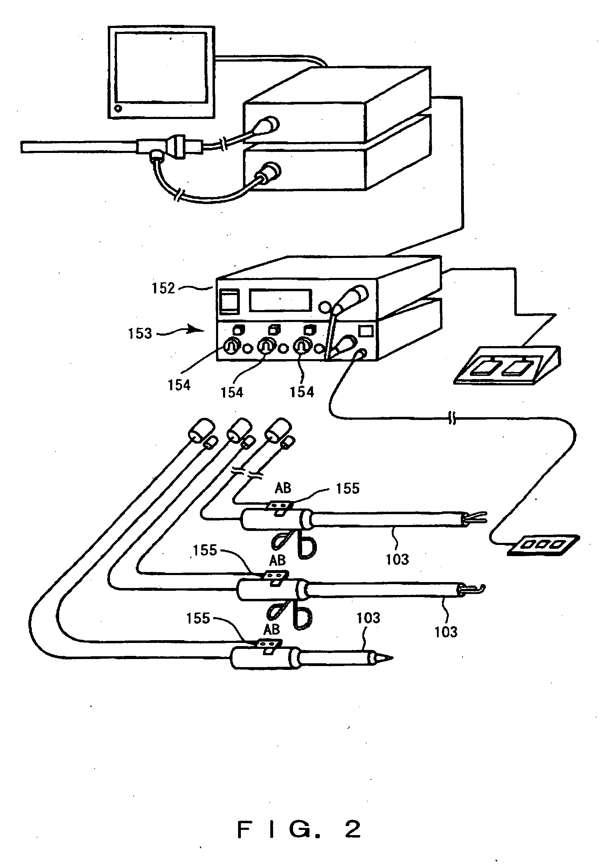

In the surgical operation system disclosed in patent document 2, a plurality of hand pieces 103 necessary for a surgical operation are connected to output ports 154 of an apparatus body 152's extension unit 153 as shown by Fig. 2. In this configuration, the extension unit 153, which outputs selectively to a plurality of output terminals, generates a drive signal for driving a hand piece 103 and selects an output operation mode by using a built-in hand switch (not shown herein) or an externally equipped hand switch 155. This configuration enables the hand piece 103 held by the surgical operator to receive a drive signal from among a plurality of hand pieces so as to easily turn on or off the output of the hand piece 103.

The surgical operation system disclosed in patent document 3 is equipped with a hand piece 93 and an apparatus body 102 as shown by Fig. 3. The hand piece 93 comprises a sensor 218 for detecting if a surgical operator is holding the hand piece 93. At the apparatus body 102, an output signal of an oscillation circuit 121 is applied through a changeover switch 122 upon activation by a sensor circuit 124 and a selection circuit 125 that receives a signal detected by the sensor 218. This configuration enables the surgical operator to perform a treatment by holding the hand piece 93 without requiring a changeover of operation by the operator, for example, by implementing the sensor 218 among a plurality of hand pieces.

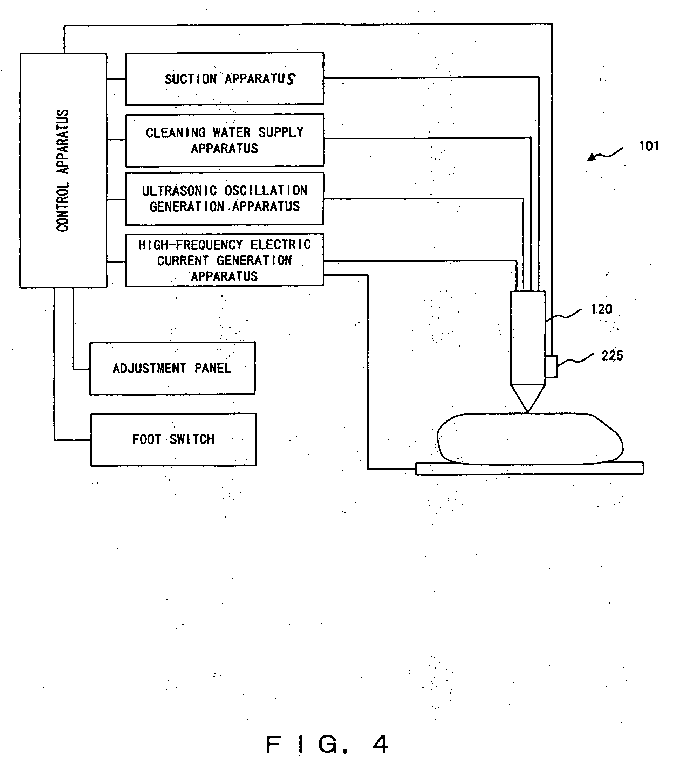

In a surgical operation-use apparatus disclosed in patent document 4, a surgical operation apparatus 101 is equipped with adjustment means for adjusting the ratio of an output of a high frequency current to that of an ultrasonic oscillation as shown by Fig. 4. In this configuration each mode is selected by a hand switch 225 equipped on a hand piece 120. This configuration makes an easy operation (please note in this specification the solitary word "operation" or "operate" means "the handling of" or "to handle" something, for example, and is meant to be differentiated from the phrase "surgical operation") at the time of a surgical operation because there is no need of an adjustment operation per output when adjusting each output.

In a surgical operation system disclosed in patent document 5, a hand piece 110 is equipped with a hand switch 119 as shown in Fig. 5. An operation of the hand switch 119 makes it possible to supply the hand piece 110 with an electric surgical knife signal by way of an electric surgical knife code 115, thereby simplifying the operation of the hand piece during a surgical operation.

The surgical operation apparatus disclosed in the above described patent document 1, however, requires a determination of a set value of the ultrasonic output in advance for shortening the time of a treatment influence. The set values, however, are different for treatment targets, and therefore it is not easy to change a set value at the applicable surgical operation apparatus if a treatment target changes during the surgical operation. This accordingly necessitates an easy change of a set value. It is further desirable to have a capability of changing not only an output of an ultrasonic output but also that of a high-frequency electric current.

The operation system and surgical operation system disclosed in the patent documents 2 and 3, respectively, are configured to select a hand piece from among a plurality of surgical operation-use hand pieces by the judgment of a surgical operator, which requires the surgical operator to change over a hand piece to a difference kind required during a surgical operation, resulting in increasing difficulties during the surgical operation.

The surgical operation apparatus disclosed in the patent document 4 proposes a selection of one out of three output modes that is adjusted for output ratios by a hand switch in order to adjust the ratio of a high frequency current output to an ultrasonic output. This configuration does not allow a surgical operator to select a discretionary output other than the three output modes provided, and does not allow the operator to change outputs during a treatment.

The surgical operation system disclosed in the patent document 5 allows a surgical operation by using an ultrasonic oscillation and an electric surgical knife, requiring a connection of a plug 115a to the hand piece 110 via a treatment-use electrode reception connector 114 at every time for providing a treatment by the electric surgical knife by receiving an electric surgical knife signal. In order to save such inconveniences, it is better to use an output changeover mechanism.
Patent document 1: Laid-Open Japanese Patent Application Publication No. 09-299381
Patent document 2: Laid-Open Japanese Patent Application Publication No. 2001-276008
Patent document 3: Laid-Open Japanese Patent Application Publication No. 2001-314411
Patent document 4: Laid-Open Japanese Patent Application Publication No. 2003-33369
Patent document 5: Laid-Open Japanese Patent Application Publication No. 2003-199762

US 2003/0040672 A1 relates to a surgical operation apparatus comprising a tip member for outputting ultrasound vibration and/or high-frequency coagulation current, a handpiece provided with the tip member, means for supplying at least the high-frequency coagulation current to the tip member, and control means for controlling the ultrasound vibration supply means and the high-frequency coagulation current supplying means. The control means includes a handswitch provided on the handpiece and a footswitch to be operated by a foot of an operator. The handswitch is provided with a cutting mode switch, a coagulation mode switch and an ultrasound output mode switch. When the cutting mode switch is turned on, a cutting mode that is a mode for mainly performing cutting of biological tissue using high-frequency cutting current and cutting mode ultrasound vibration is selected. When the coagulation mode switch is turned on, a coagulation mode, that is a mode for mainly performing coagulation and hemostasis of blood or biological tissue using high-frequency coagulation current and coagulation mode ultrasound vibration is selected. In addition, when the ultrasound output mode switch is turned on, an ultrasound vibration mode for fragmenting tissue and aspirating fragmented tissue only by ultrasound vibration is selected.

US 2002/0115917 A1 discloses a medical treatment system comprising a plurality of medical treatment equipments. An ultrasound therapeutic instrument for coagulation/incision treatment of a diseased part using ultrasound vibrations as provided. Further, a high-frequency therapeutic instrument performing coagulation/incision treatment by passing high-frequency electric current to the diseased part is provided.

### Disclosure of Invention

The present invention provides an ultrasonic surgical operation system according to claim 1.

The present invention provides an ultrasonic surgical operation system allowing a surgical operator to operate a switch connected to an apparatus body, or a hand switch (i.e., a switch terminal) built-in or freely detachable to a hand piece according to a judgment of the surgical operator, thereby enabling an easy changeover of output modes prior to and during a treatment, an elimination of operational difficulties in a surgical operation room and for the surgical operator, a reduction in equipment costs, an effective use of a space in a surgical operation room and of an improved efficiency of a surgical operation.

An exemplary ultrasonic surgical operation system comprises a hand piece having a probe to which an ultrasonic oscillation and a high-frequency electric current are transmitted; an ultrasonic oscillation drive unit capable of generating and outputting to the hand piece an ultrasonic oscillation signal in order to ultrasonically oscillate the probe, and capable of changing output modes of the ultrasonic oscillation; a high-frequency electric current output unit capable of outputting the high-frequency electric current to the hand piece and capable of changing output modes of the high-frequency electric current; a first output instruction unit for instructing the ultrasonic oscillation drive unit to output the ultrasonic oscillation signal; a second output instruction unit for instructing the high-frequency electric current output unit to output the high-frequency electric current; a changeover instruction unit for instructing the change over of the output modes and output values of at least one of the ultrasonic oscillation drive unit and high-frequency electric current output unit; and a control unit, being one for changing over the output modes and output values of at least one of the ultrasonic oscillation drive unit and high-frequency electric current output unit based on the instruction from the changeover instruction unit, for selectively changing over either one of the output modes and output values according to the instruction by the first or second output instruction unit.

A exemplary usage method of an ultrasonic surgical operation system, not forming part of the invention, is disclosed that includes a hand piece having a probe to which an ultrasonic oscillation and a high-frequency electric current are transmitted, an ultrasonic oscillation drive unit capable of generating and outputting to the hand piece an ultrasonic oscillation signal in order to ultrasonically oscillate the probe and capable of changing output modes of the ultrasonic oscillation, and a high-frequency electric current output unit capable of outputting the high-frequency electric current to the hand piece and changing output modes of the high-frequency electric current and is capable of changing over the output modes of at least one of the high-frequency electric current and ultrasonic oscillation prior to or during a treatment; and changing output values of the high-frequency electric current and ultrasonic oscillation in the post-change output mode during the treatment.

### Brief Description of Drawings

Fig. 1 is a diagram showing a configuration of a conventional surgical operation apparatus;
Fig. 2 is a diagram showing a configuration of a conventional surgical operation apparatus;

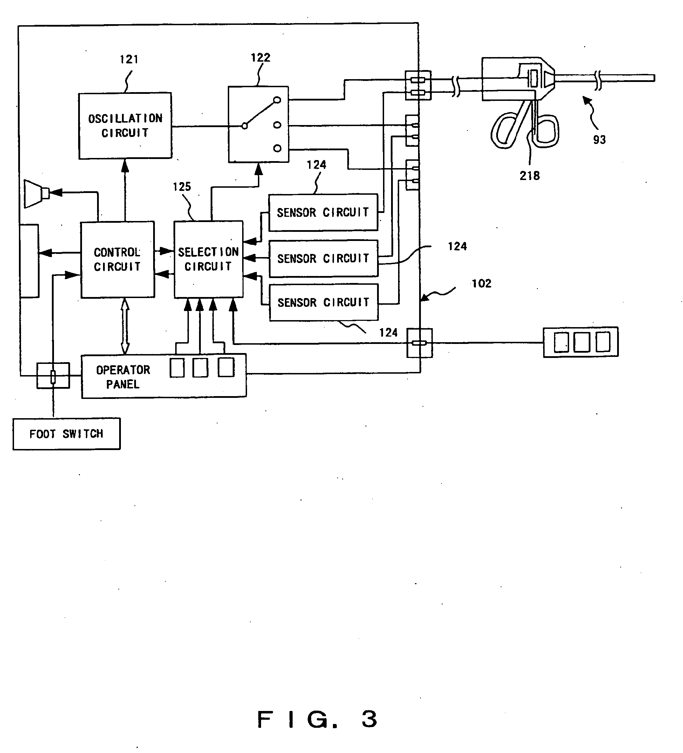

Fig. 3 is a diagram showing a configuration of a conventional surgical operation apparatus;
Fig. 4 is a diagram showing a configuration of a conventional surgical operation apparatus;

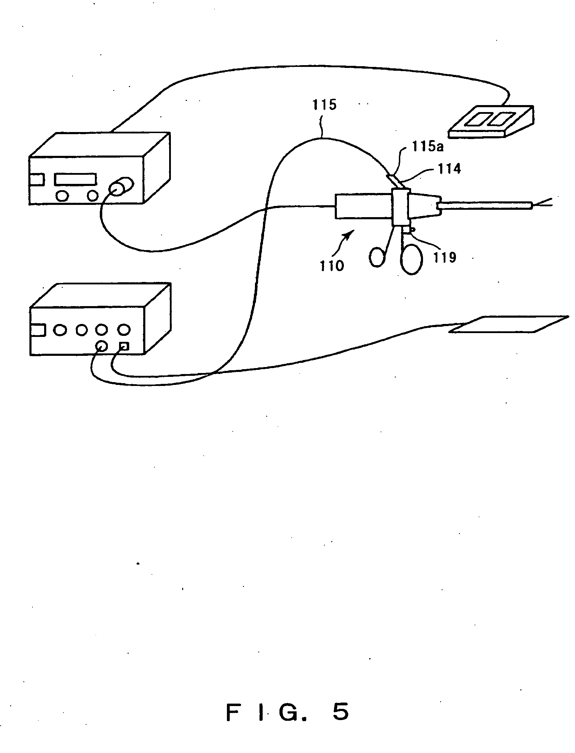

Fig. 5 is a diagram showing a configuration of a conventional surgical operation apparatus;
Fig. 6 is a diagram illustrating a configuration of an ultrasonic surgical operation system according to a present embodiment;
Fig. 7 is a diagram showing block configurations of an ultrasonic oscillation drive apparatus and a high-frequency electric current output apparatus within an ultrasonic surgical operation system;
Fig. 8 is a diagram exemplifying a block configuration when an ultrasonic oscillation drive apparatus communicates with a high-frequency electric current output apparatus within an ultrasonic surgical operation system;
Fig. 9 is a diagram showing a flow chart at the time of a transition of output modes in Standby states of an ultrasonic oscillation drive apparatus 2 and a high-frequency electric current output apparatus 1 both according to a present embodiment;
Fig. 10 is a diagram showing flow charts at the time of a transition of output modes during an ultrasonic output, with (a) showing the case of an initial state being set to an ultrasonic output mode and (b) showing the case of an initial state being set to a maximum ultrasonic output mode; and
Fig. 11 is a diagram showing flow charts at the time of a transition of output modes during a high-frequency electric current output, with (a) showing the case of an initial state being an incision-use high-frequency electric current output mode and (b) showing the case of an initial state being a coagulation-use high-frequency electric current output mode.

### Best Mode for Carrying Out the Invention

The present invention is an ultrasonic surgical operation system having a first output switch (e.g., a foot switch) used to issue an ultrasonic output instruction, a second switch (described below as a switch terminal 14) used to issue a high-frequency output instruction and a changeover switch (described below as a switch terminal 13) used to change output modes and output values. The ultrasonic surgical operation system according to the present invention is configured to change over output modes if a changeover switch is pressed in a state of neither the first output switch nor the second output switch, and will change over output values if the changeover switch is pressed in a state of either the first output switch or the second output switch (i.e., the changeover switch has two functions which are selectively used).

The following is a detailed description of the preferred embodiment of the present invention by referring to the accompanying drawings.

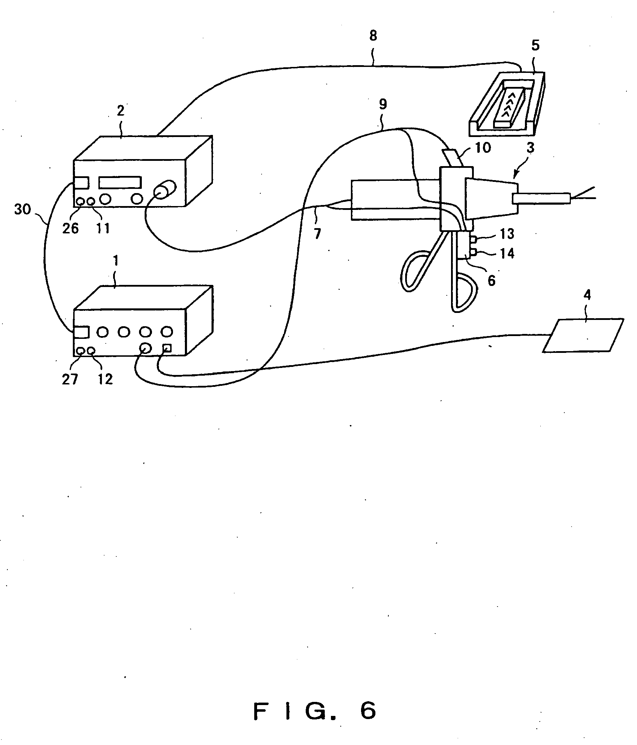

Fig. 6 is a diagram illustrating a configuration of an ultrasonic surgical operation system according to a present embodiment. The ultrasonic surgical operation system primarily comprises a high-frequency electric current output apparatus 1, an ultrasonic oscillation drive apparatus 2, a hand piece 3, a counter electrode plate 4 and a foot switch 5.

The ultrasonic oscillation drive apparatus 2 is connected to the foot switch 5 by way of a foot switch cable 8. The foot switch 5 is a switch for turning on and off an output of an ultrasonic oscillation. An operator panel on the front of the ultrasonic oscillation drive apparatus 2 is equipped with various connectors, a display unit, a set changeover switch 11 and a button 26.

The high-frequency electric current output apparatus 1 is connected to the counter electrode plate 4 . The operator panel on the front of the high-frequency electric current output apparatus 1 is equipped with switches, such as various connectors, a display unit, a set changeover switch 12 and a button 27. The hand piece 3 is equipped with an electrode unit 10 and a hand switch 6 which is equipped with switch terminals 13 and 14.

The hand piece 3 is connected to high-frequency electric current output apparatus 1 by an active cord 9 by way of the electrode unit 10. The hand piece 3 is also connected to the ultrasonic oscillation drive apparatus 2 by way of a freely detachable connector attach-detach cable 7. The hand switch 6 is equipped on the hand piece 3 either by being built in or freely detachable and inserted therein. The hand switch 6 is further connected to the high-frequency electric current output apparatus 1 by way of the active cord 9. Furthermore, the hand switch 6 is connected to the ultrasonic oscillation drive apparatus 2 by way of the connector attach-detach cable 7.

The hand piece 3 houses an ultrasonic transducer. The configuration is such that a drive signal generated in the ultrasonic oscillation drive apparatus 2 is output to the ultrasonic transducer housed by the hand piece 3, and that the ultrasonic transducer housed by the hand piece 3 converts the input drive signal into a mechanical oscillation so as to produce an ultrasonic oscillation. A base part of a probe transmitting the ultrasonic oscillation is connected to the hand piece 3. The ultrasonic oscillation generated by the ultrasonic transducer housed by the hand piece 3 is transmitted to the probe. A tip part of the probe is disposed for providing a treatment target with a treatment by using the ultrasonic oscillation transmitted from the base part to the tip part of the probe.

The high-frequency electric current output apparatus 1 is configured to generate an electric surgical knife signal for the purpose of causing a hemostat, for example, by applying a high-frequency electric current to a living body tissue from the tip of the hand piece 3 by way of the active cord 9.

The switch terminals 13 and 14 may use a push button switch, a pressure switch, or an optical switch, for example. The switch terminal 14 is configured to turn on and off a high-frequency electric current.

The switch terminal 13 is a switch configured to change over output modes of the high-frequency electric current output apparatus 1 and ultrasonic oscillation drive apparatus 2. The switch terminal 13 is equipped with two changeover functions. A first of the two functions is one for changing over the output modes of the high-frequency electric current output apparatus 1 and ultrasonic oscillation drive apparatus 2. The second is the function for changing the respective sizes of output values of a high-frequency electric current or an ultrasonic oscillation in the post-change output mode. These functions are further explained below. Note that the present embodiment is configured to make the switch terminal 13 comprise these two functions, each function, however, may be implemented with individual independent switches. Also, the switch terminals 13 and 14 may use foot switches, or switches built in or freely detachable to the hand piece.

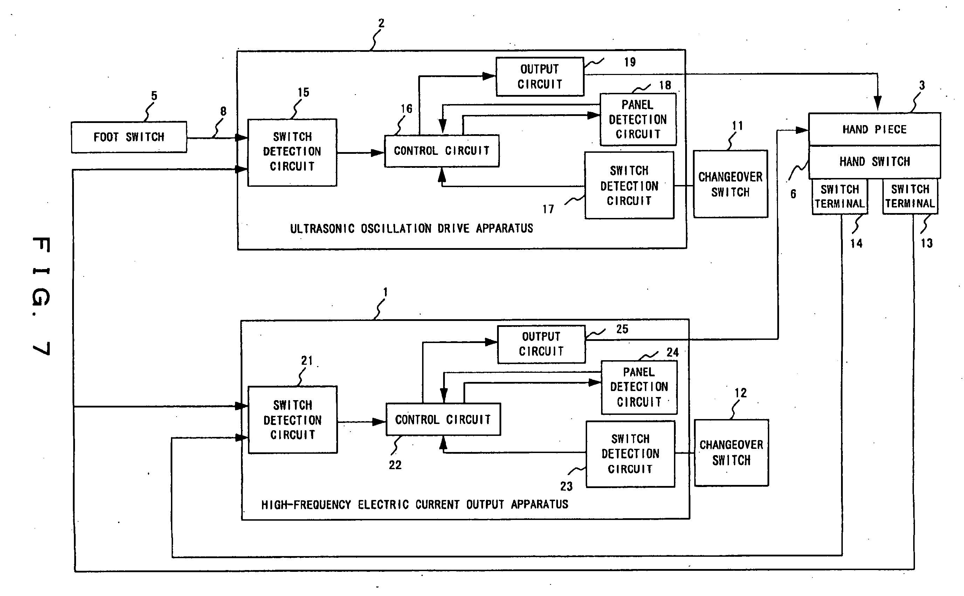

Fig. 7 is a diagram showing block configurations of an ultrasonic oscillation drive apparatus and a high-frequency electric current output apparatus within an ultrasonic surgical operation system. The ultrasonic oscillation drive apparatus 2 comprises a changeover switch 11, switch detection circuits 15 and 17, a control circuit 16, a panel detection circuit 18 and an output circuit 19.

The high-frequency electric current output apparatus 1 comprises a changeover switch 12, switch detection circuits 21 and 23, a control circuit 22, a panel detection circuit 24 and an output circuit 25. Signals from the foot switch 5 and the switch terminal 13 equipped on the hand switch 6 are transmitted to the control circuit 16 by way of the switch detection circuit 15. A changeover signal from the changeover switch 11 is detected by the switch detection circuit 17 and transmitted to the control circuit 16. The panel detection circuit 18 detects an operation state of the operator panel on the front of the ultrasonic oscillation drive apparatus 2 based on an instruction from the control circuit 16. Output setup information detected by the panel detection circuit 18 is transmitted to the control circuit 16. Having received a control signal from the control circuit 16, the output circuit 19 outputs a signal for an ultrasonic output to the hand piece 3.

Signals from the switch terminals 13 and 14, which are equipped on the hand switch 6, are output to the control circuit 22 by way of the switch detection circuit 21. A changeover signal from the changeover switch 12 is also transmitted to the control circuit 22 by way of the switch detection circuit 23.

The panel detection circuit 24 detects an operation state of the operator panel on the front of the high-frequency electric current output apparatus 1. Output setup information detected by the panel detection circuit 24 is output to the control circuit 22 based on an instruction therefrom. Having received a control signal from the control circuit 22, the output circuit 25 outputs a signal for high-frequency electric current output to the hand piece 3.

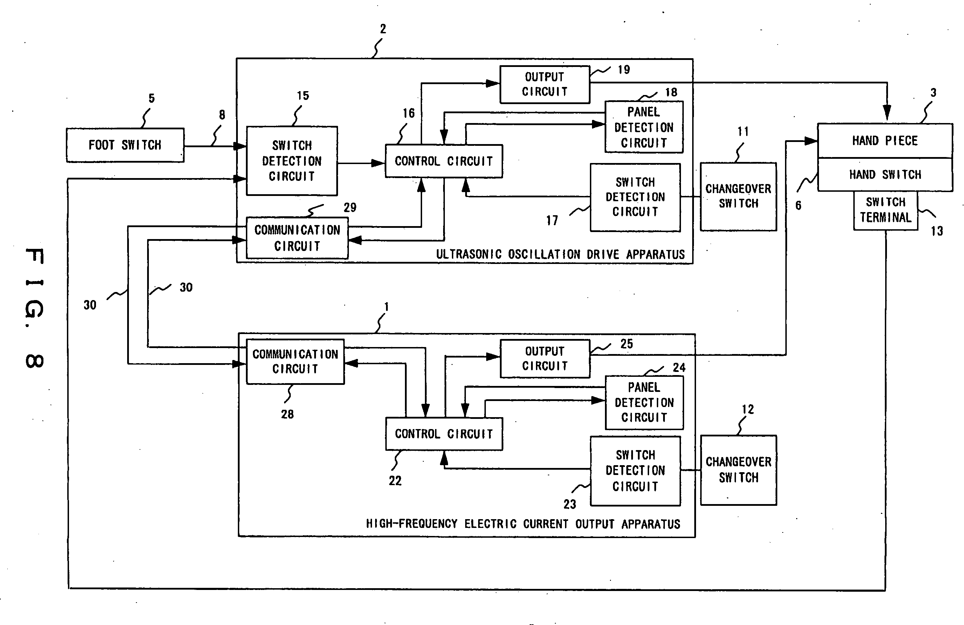

Fig. 8 is a diagram exemplifying a block configuration when an ultrasonic oscillation drive apparatus communicates with a high-frequency electric current output apparatus 1 within an ultrasonic surgical operation system. Being a modified embodiment of Fig. 7, the configuration shown by Fig. 8 has the addition of communication circuits 28 and 29, and a communication cable 30, and the switch terminal 14 is eliminated. A further description of Fig. 8 is below.

The high-frequency electric current output apparatus 1 and ultrasonic oscillation drive apparatus 2 are each equipped with communication circuit 28 and 29, respectively, which are interconnected by communication cable 30.

In this configuration, a signal of the switch terminal 13 is detected by the switch detection circuit 15 of the ultrasonic oscillation drive apparatus 2 by way of the communication cable 30, and is transmitted to the high-frequency electric current output apparatus 1 via communication circuits 28 and 29. The signal of the switch terminal 13 is mutually transmittable between the high-frequency electric current output apparatus 1 and ultrasonic oscillation drive apparatus 2 by way of the communication cable 30. The signal of the switch terminal 14 is also mutually transmittable between the high-frequency electric current output apparatus 1 and ultrasonic oscillation drive apparatus 2 by way of the communication cable 30. A signal of the foot switch 5 is likewise mutually transmittable between the high-frequency electric current output apparatus 1 and ultrasonic oscillation drive apparatus 2 by way of the communication cable 30.

Therefore, in the case of equipping the foot switch 5 in place of a switch terminal 14 as shown in Fig. 8, turning on and -off of the output of the ultrasonic oscillation drive apparatus 2 and high-frequency electric current output apparatus 1 can be controlled by the foot switch 5. Alternatively, in the case of equipping the switch terminal 14 in place of a foot switch 5, turning on and -off of the output of the ultrasonic oscillation drive apparatus 2 and high-frequency electric current output apparatus 1 can be controlled by the switch terminal 14.

The following description is a comparison of the difference in function between the absence and presence of a communication function in the configurations shown by Figs. 7 and 8. In the absence (shown by Fig. 7) of communications between the high-frequency electric current output apparatus 1 and ultrasonic oscillation drive apparatus 2 by way of a communication cable 30, a first output switch (i.e., a foot switch) needs to be pressed in order to output an ultrasonic wave, and a second output switch (i.e., a switch terminal 14) needs to be pressed in order to output a high-frequency wave. As shown in Fig. 7, in the case of an ultrasonic wave output mode being set up, nothing is output if the second output switch for outputting a high-frequency wave is pressed in. Additionally, in the case of a high-frequency wave output mode being set up, nothing is output if the first output switch for outputting an ultrasonic wave is pressed. Furthermore, two output switches need to be pressed respectively in order to output simultaneously an ultrasonic wave and a high-frequency wave in the ultrasonic wave and high-frequency wave output mode.

As shown by Fig. 8, in the configuration of the high-frequency electric current output apparatus 1 communicating with the ultrasonic oscillation drive apparatus 2 by way of a communication cable 30, signals input to the respective control units of the high-frequency electric current output apparatus 1 and ultrasonic oscillation drive apparatus 2 are mutually transmitted, and instruction signals from the changeover switch (i.e., the switch terminal 13) and the first and second output switches are accordingly transmitted to the other apparatus by way of the communication cable 30 (Fig. 8 exemplifies a case of equipping the first output switch in place of equipping the second output switch) . In such a configuration, a pressing of either output switch outputs an ultrasonic wave or a high-frequency wave depending on which output mode is set (i.e., an ultrasonic wave is output in the ultrasonic wave output mode, a high-frequency wave is output in the high-frequency wave output mode, and an ultrasonic wave and a high frequency wave are simultaneously output just by pressing one switch in the ultrasonic wave and high-frequency wave output mode). This eliminates a necessity of two output switches, and instead reduces to just one output switch as shown in Fig. 8.

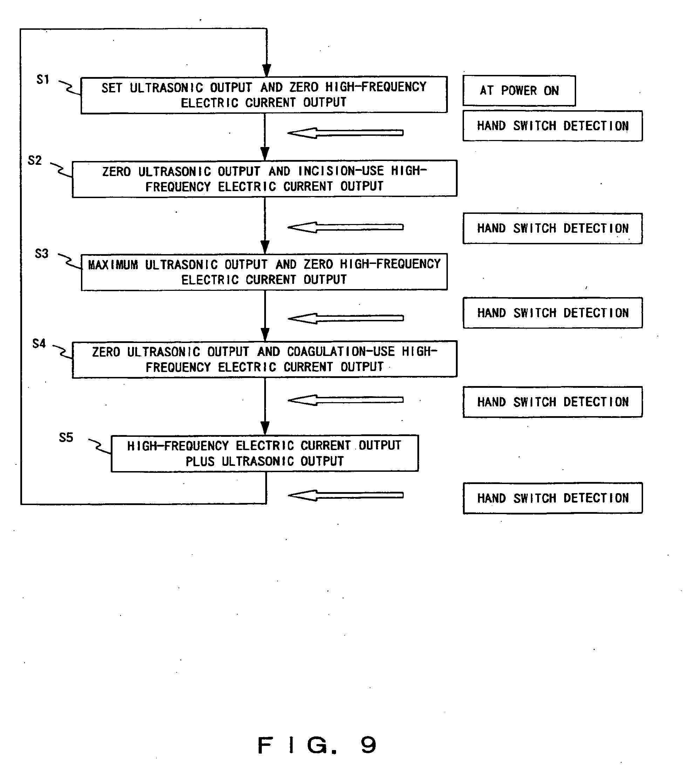

Fig. 9 is a diagram showing a flow chart at the time of a transition of output modes in Standby states of an ultrasonic oscillation drive apparatus 2 and a high-frequency electric current output apparatus 1 both according to the present embodiment. The configuration shown by Fig. 9 uses the first function of the switch terminal 13 (i.e. , the function of changing over output modes of the high-frequency electric current output apparatus 1 and ultrasonic oscillation drive apparatus 2) .

The ultrasonic oscillation drive apparatus 2 is set up with four modes, i.e., "set ultrasonic output mode", "maximum ultrasonic output mode", "high-frequency electric current output plus ultrasonic output mode" and "zero output mode", for example. Note that the output modes are not limited to these four modes, and additional modes may be implemented. Incidentally, what is different relative to the ultrasonic outputs (i.e., "set ultrasonic output mode" and "maximum ultrasonic output mode") of the modes regarding the ultrasonic output is an output value of an output ultrasonic wave.

Meanwhile, the high-frequency electric current output apparatus 1 is set up with four modes, i.e., "incision-use high-frequency electric current output mode", "coagulation-use high-frequency electric current output mode", "high-frequency electric current output plus ultrasonic output mode" and "zero output mode", for example. Note that the output modes are not limited to these four modes, and additional modes may be implemented. Incidentally, what is different relative to the high-frequency electric current output (i.e., "incision-use high-frequency electric current output" and "coagulation-use high-frequency electric current output") in the modes relating to the high-frequency electric current output is an output value of an high-frequency electric current output.

In the ultrasonic oscillation drive apparatus 2 and high-frequency electric current output apparatus 1, a pressing of Ready buttons 26 and 27 of the respective apparatuses makes it possible to change over between a Ready state and a Standby state. A pressing of the setup changeover switch 11 at the time of the Ready state in the ultrasonic oscillation drive apparatus 2 performs a transition between the two output modes, i.e., "set ultrasonic output mode" or "high-frequency electric current output plus ultrasonic output mode". This makes a display unit of the ultrasonic oscillation drive apparatus 2 display the fact of a transition of the output modes and makes a speaker equipped thereon notify the fact of a transition of the output modes by way of a voice guide (or a buzzer tone, et cetera) . In this event, an ultrasonic output value for each mode is determined.

The high-frequency electric current output apparatus 1 performs a transition among three output modes, i.e., "incision-use high-frequency electric current output mode", "coagulation-use high-frequency electric current output mode" and "high-frequency electric current output plus ultrasonic output mode" when the setup changeover switch 12 is pressed at the time of the Ready state. This makes a display unit of the high-frequency electric current output apparatus 1 display the fact of a transition of the output modes and a speaker equipped thereon notify the fact of a transition of the output modes by way of a voice guide (or a buzzer tone, et cetera). In this event, a high-frequency electric current output set value for each mode is determined. For example, the high-frequency electric current output in the "high-frequency electric current output plus ultrasonic output mode" is used for coagulation.

Respective outputs are enabled at the time of Standby states of the ultrasonic oscillation drive apparatus 2 and high-frequency electric current output apparatus 1, and a display and a voice guide (or a buzzer tone, et cetera) are output at each of the apparatuses during the output of the aforementioned respective outputs. In the example shown by Fig. 9, the ultrasonic oscillation drive apparatus 2 is in the "set ultrasonic output mode", while the high-frequency electric current output apparatus 1 is in the zero high-frequency electric current output mode, at the time of turning the power on (S1). A pressing of only the switch terminal 13 equipped on the hand switch 6 (that is, neither the foot switch 5 nor the switch terminal 14 of the hand switch 6 is pressed) makes the output mode shift to the "zero ultrasonic output and an incision-use high-frequency electric current output" mode (S2). Then, a pressing of only the switch terminal 13 makes the output mode shift to the "maximum ultrasonic output and zero high-frequency electric current output" mode (S3). Then, a pressing of only the switch terminal 13 makes the output mode shift to the "zero ultrasonic output and incision-use high-frequency electric current output" mode (S4) . Then, a pressing of only the switch terminal 13 makes the output mode shift to the "high-frequency electric current output plus ultrasonic output" mode (S5). Then, a pressing of only the switch terminal 13 makes the output mode shift again to the "set ultrasonic wave output and zero high-frequency electric current output" mode (S1).

As such, a pressing of only the switch terminal 13 equipped on the hand switch 6 (that is, neither the foot switch 5 nor the switch terminal 14 of the hand switch 6 is pressed) makes the output mode sequentially shift in addition to a display and a voice guide (or a buzzer tone, et cetera) at everypressing of the relevant switch. Correspondingly, every transition of output modes makes the display unit of the ultrasonic oscillation drive apparatus 2 or high-frequency electric current output apparatus 1 display the fact of a transition of the output modes and makes the speaker equipped on the ultrasonic oscillation drive apparatus 2 or high-frequency electric current output apparatus 1 notify the fact of a transition of the output modes by a voice guide (or a buzzer tone, et cetera).

According to the present embodiment, the sequence of transitions of output modes is one shown in Fig. 9 (i.e., S1→ S2→ S3→ S4→ S5→ S1→ S2 and so on), in which the output modes of the ultrasonic oscillation drive apparatus 2 and high-frequency electric current output apparatus 1 shift simultaneously. Note that the changeover sequence of the output modes is not limited by this configuration of the present embodiment. Additionally, a selected mode name is displayed by the display units equipped on the high-frequency electric current output apparatus 1 and ultrasonic oscillation drive apparatus 2.

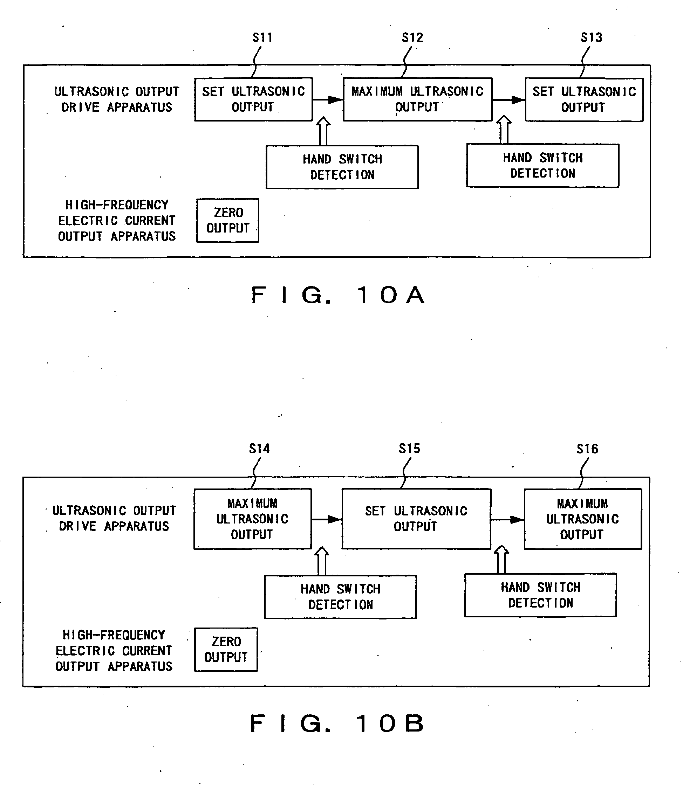

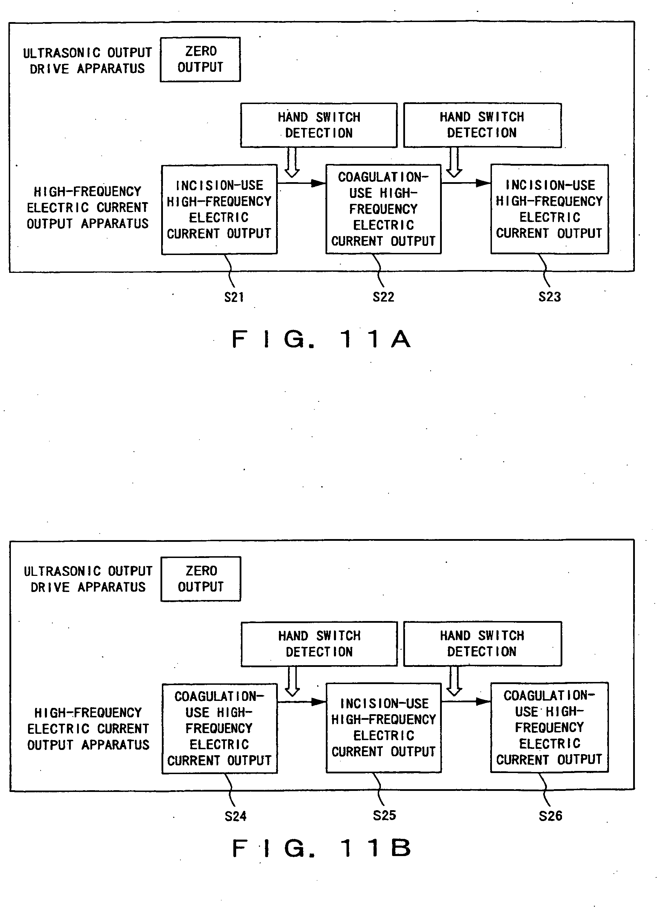

The next description is of a second function of the switch terminal 13 (i.e. , the function of changing a size of an output value of a high-frequency electric current or an ultrasonic oscillation in a selected output mode by being changed over in Fig. 9) by using Figs. 10 and 11.

Fig. 10 is a diagram showing flow charts at the time of a transition of output modes within an ultrasonic output. Corresponding to a pressing of the foot switch 5, an ultrasonic output is started while a display and a voice guide (or a buzzer tone, et cetera) matching with each output mode are presented. Note that an output mode described in relation to Fig. 10 is defined as an output state based on a size of an output value.

Below is the description of the configurations shown in Fig. 10 (a). First, a pressing of the switch terminal 13 during an ultrasonic output in the state of the foot switch 5 being pressed makes a transition from a set ultrasonic output mode (S11) to a maximum ultrasonic output mode (S12) without stopping an ultrasonic output. In this event, the post-transition mode is displayed and notified by a voice guide (or a buzzer tone, et cetera) responding to the mode transition.

A further pressing of the switch terminal 13 (with the foot switch 5 being pressed) makes another transition from the maximum ultrasonic output mode (S12) to a set ultrasonic output mode (S13), and the display of the ultrasonic oscillation drive apparatus 2 also makes a transition. In this event, the post-transition mode is displayed and notified by a voice guide (or a buzzer tone, et cetera) corresponding to the mode transition.

The next description is of the case of Fig. 10 (b). First, a pressing of the switch terminal 13 during an output in the state of the foot switch 5 being pressed makes a transition from a maximum ultrasonic output mode (S14) to a set ultrasonic output mode (S15) without the ultrasonic output stopping. In this event, the post-transition mode is displayed and notified by a voice guide (or a buzzer tone, et cetera) corresponding to the mode transition.

A further pressing of the switch terminal 13 (with the foot switch 5 being pressed) makes another transition from the set ultrasonic output mode (S15) to a maximum ultrasonic output mode (S16), and the display of the ultrasonic oscillation drive apparatus 2 also makes a transition. In this event, the post-transition mode is displayed and notified by a voice guide (or a buzzer tone, et cetera) corresponding to the mode transition.

Note that, when releasing the foot switch 5 for stopping the output, the output mode returns to an initial state (i.e., to the set ultrasonic output mode in the case of Fig. 10 (a) ; and to the maximum ultrasonic output mode in the case of Fig. 10 (b)). In this event, the display of the ultrasonic oscillation drive apparatus 2 also makes a transition to the post-return mode.

When a surgical operator applies a gradual effect of a frictional heat to a living tissue of a patient for example, he starts it in a set ultrasonic output mode, followed by pressing the switch terminal 13 of the hand switch 6 in the state of the foot switch 5 being pressed at the time of starting an incision (refer to Fig. 10 (a) ) . This enables him to perform a rapid incision by making a transition to a maximum ultrasonic output mode.

When the surgical operator applies a rapid effect of a frictional heat to a living tissue, he starts it in amaximumultrasonic output mode at the time of starting an incision, followed by pressing the switch terminal 13 of the hand switch 6 in the state of the foot switch 5 being pressed for making a transition to a set ultrasonic output mode according to his judgment, thereby performing a normal incision (refer to Fig. 10 (b)). This configuration enables a shortening of an influence time.

Fig. 11 is a diagram showing flow charts at the time of a transition of output modes during a high-frequency electric current output. A pressing of the switch terminal 14 equipped on the hand switch 6 starts a high-frequency electric current output along with a display in the display unit and a voice guide (or a buzzer tone, et cetera) that matches each output mode. Note that an output mode described in relation to Fig. 11 is defined as an output state based on a size of an output value.

The next description is of the configuration of Fig. 11 (a). First, a further pressing of the switch terminal 13 during an output of a high-frequency electric current output in the state of the switch terminal 14 equipped on the hand switch 6 being pressed makes a transition from an incision-use high-frequency electric current output mode (S21) to a coagulation-use high-frequency electric current output mode (S22) without the high-frequency electric current output stopping. In this event, the post-transition mode is displayed by the display unit and notified by a voice guide (or a buzzer tone, et cetera) corresponding to the mode transition.

A further pressing of the switch terminal 13 (with the foot switch 14 being pressed) makes another transition from the coagulation-use high-frequency electric current output mode (S22) to the incision-use high-frequency electric current output mode (S23) and the display of the high-frequency electric current output apparatus 1 also makes a transition. In this event, the post-transition mode is displayed by the display unit and notified by a voice guide (or a buzzer tone, et cetera) corresponding to the mode transition.

The next description is of the configuration of Fig. 11 (b). First, a further pressing of the switch terminal 13 during an output of a high-frequency electric current output in the state of the terminal 14 equipped on the hand switch 6 being pressed makes a transition from a coagulation-use high-frequency electric current output mode (S24) to an incision-use high-frequency electric current output mode (S25) without the high-frequency electric current output stopping. In this event, the post-transition mode is displayed by the display unit and notified by a voice guide (or a buzzer tone, et cetera) corresponding to the mode transition.

A further pressing of the switch terminal 13 (with the switch terminal 14 being pressed) makes another transition from the incision-use high-frequency electric current output mode (S25) to the coagulation-use high-frequency electric current output mode (S26), and the display of the high-frequency electric current output apparatus 1 also makes a transition. In this event, the post-transition mode is displayed by the display unit and notified by a voice guide (or a buzzer tone, etcetera) corresponding to the mode transition.

Incidentally, when releasing the switch terminal 14 of the hand switch 6 for stopping the output, the output mode returns to an initial state (i.e., an incision-use high-frequency electric current output mode in the case of Fig. 11 (a); and a coagulation-use high-frequency electric current output mode in the case of Fig. 11 (b)) and the display of the high-frequency electric current output apparatus 1 also makes a transition to that of the post-return mode.

Therefore, it is possible to make quick transitions between the incision-use high-frequency electric current output mode and coagulation-use high-frequency electric current output mode by tracking changeover instructions of a surgical operator during a high-frequency wave output, thereby shortening a treatment time.

A setup of a "high-frequency electric current output plus ultrasonic wave output" mode enables an ultrasonic output by pressing the foot switch 5 and a high-frequency electric current output by pressing the switch terminal 14 of the hand switch 6, thereby enabling a simultaneous output. An output sequence of the ultrasonic output and high-frequency electric current output is not apparently limited.

In the "high-frequency electric current output and ultrasonic output" mode, a display and a voice guide (or a buzzer tone, et cetera) at the time of a set ultrasonic output is carried out when outputting only an ultrasonic wave, and a display and a voice guide (or a buzzer tone, et cetera) at the time of a coagulation-use high-frequency electric current output is carried out when outputting only a high-frequency electric current output. When outputting both a high-frequency electric current output and an ultrasonic output simultaneously, a display and a voice guide (or a buzzer tone, et cetera) during the output are different from each of the above described display and voice guide (or a buzzer tone, et cetera) . In the case of the high-frequency electric current output apparatus 1 and ultrasonic oscillation drive apparatus 2 being interconnected by way of the communication cable 30 for outputting, it is possible to change output values of the high-frequency electric current output and ultrasonic output by using either of the switch terminal 14 of the hand switch 6 and the foot switch 5 as described in association with Fig. 8. This allows the equipment to comprise only one of either of the two switches, i.e., the switch terminal 14 of the hand switch 6 or the foot switch 5. In the case of Fig. 8 for example, a pressing of the foot switch 5 causes an ultrasonic wave to be output at the time of an ultrasonic output mode, a pressing of the foot switch 5 causes a high-frequency wave to be output at the time of a high frequency output mode, and a pressing of the foot switch 5 causes an ultrasonic wave and a high-frequency wave to be simultaneously output at the time of an "ultrasonic output and high frequency output" mode.

The present embodiment is configured to enable a surgical operator to easily change the output modes of a high-frequency electric current or an ultrasonic oscillation output during a treatment by using the surgical operator's judgment by operating a hand switch equipped on a hand piece. Also enabled is an easy and quick changeover from a high output to a low output during a treatment, thereby making it possible to carry out a safe and effective surgical operation.

Furthermore, the capability to change over the output modes without requiring the surgical operator to switch hand pieces avoids the problems caused by switching the hand pieces and also prevents the interruption of a surgical operation. Therefore it is possible to shorten the time and carry out a more efficient surgical operation.

An output change from a high output to a low output makes it possible to quickly elongate the influence time to a tissue by heat, and, conversely, perform a tissue incision at a discretionary time judged by the surgical operator as a result of the surgical operator sufficiently influencing the tissue by a frictional heat at the time of a treatment, followed by changing from low output to a high output at the time when he wants to carry out the tissue incision.

Meanwhile, two output apparatuses, i.e., an ultrasonic output and a high-frequency electric current output, integrated as a single system enables the reduction of the number of foot switches and the associated reduction of equipment costs, the elimination of complex space within a surgical operation room, and an increase in the efficiency of a surgical operation.

As described above, the present invention enables a surgical operator to operate a switch connected to an apparatus body or a hand switch, built in or freely detachable attached to a hand piece at a discretion of the surgical operator, thereby enabling an easy changeover of output modes prior to or during a treatment, the elimination of cumbersome equipment used in the surgical operating room, and allows a surgical operator, as a result of reducing the number of foot switches by two output modes using a single system and the associated reduction of equipment costs, to have a more effective and efficient use of surgical operation room space, and finally it provides for greater efficiency in a surgical operation.

## Claims

1. An ultrasonic surgical operation system, comprising:
a hand piece having (3) a probe to which an ultrasonic oscillation and a high-frequency electric current are transmitted;
an ultrasonic oscillation drive unit (19) capable of generating and outputting to the hand piece an ultrasonic oscillation signal in order to ultrasonically oscillate the probe, and changing output modes of the ultrasonic oscillation;
a high-frequency electric current output unit (25) capable of outputting the high-frequency electric current to the hand piece and changing output modes of the high-frequency electric current;
a first output instruction unit (5) for instructing the ultrasonic oscillation drive unit to output the ultrasonic oscillation signal;
a second output instruction unit (14) for instructing the high-frequency electric current output unit to output the high-frequency electric current;
a changeover instruction unit (13) for instructing to change over the output modes and output values of at least one of the ultrasonic oscillation drive unit (19) and high-frequency electric current output unit (25); and
a control unit (16, 22) that is adapted to change over the output modes and output values of at least one of the ultrasonic oscillation drive unit (19) and high-frequency electric current output unit (25) based on the instruction from the changeover instruction unit (13), for selectively changing over either one of the output modes and output values according to the instruction by the first (5) or second output instruction unit (14),
wherein said control unit (16, 22) is further adapted to change over said output modes of said high-frequency electric current (25) and/or said ultrasonic oscillation (19) based on said instruction from said changeover instruction unit (13) if there is no output instruction from either of said first (5) and second (14) output instruction units, or to change output values of at least either of the high-frequency electric current and ultrasonic oscillation based on the instruction from the changeover instruction unit (13) if there is the output instruction from either of said first (5) and second (14) output instruction units, and
said changeover instruction unit (13) is formed of a single switch equipped on the hand piece (3).

2. The ultrasonic surgical operation system according to claim 1, wherein
said first output instruction unit (5), said second output instruction unit (14), and said changeover instruction unit (13) output respective instruction signals to the said ultrasonic oscillation drive unit (19), said high-frequency electric current output unit (25) and said control unit (16, 22), for said instruction to them;
the control unit comprises
one or more instruction signal detection units for detecting the instruction signal output from the first output instruction unit (5), second output instruction unit (14), or changeover instruction unit (13), and
one or more changeover control units (16, 22) for changing over said output modes of at least either one of said high-frequency electric current and said ultrasonic oscillation based on the instruction signal detected by the instruction signal detection unit (15, 21), and changing over said output values of at least either one of the high-frequency electric current and ultrasonic oscillation in the changed-over output mode; and
wherein the changeover control unit (16, 22) is adapted to change over the output modes based on an instruction signal if only the instruction signal from the changeover instruction unit is detected by the instruction signal detection unit (15, 21), or to change over the output values based on an instruction signal if the instruction signal from either one of the first and second output instruction units and the instruction signal from the changeover instruction unit are detected by the instruction signal detection unit.

3. The ultrasonic surgical operation system according to claim 1, wherein
said changeover instruction unit is a switch (13) built in said hand piece (3).

4. The ultrasonic surgical operation system according to claim 1, wherein
said first output instruction unit (5) is a foot switch or a switch (14) built in, or freely detachably attached, to said hand piece(3).

5. The ultrasonic surgical operation system according to claim 1, wherein
said second output instruction unit (14) is a foot switch or a switch built in, or freely detachably attached, to said hand piece (3).

6. The ultrasonic surgical operation system according to claim 1, wherein
said output modes possessed by said ultrasonic oscillation drive unit (19) at least include either a set ultrasonic output mode for outputting an ultrasonic oscillation of a set output value, a maximum ultrasonic output mode for outputting an ultrasonic oscillation of an upper limit output value, or a zero output mode;
said output modes possessed by said high-frequency electric current output unit (25) at least include either an incision-use high-frequency electric current output mode, a coagulation-use high-frequency electric current output mode, or a zero output mode, and
said output modes allowed for changing over by said control unit (16, 22) are output modes constituted by combinations of the output modes possessed by the ultrasonic oscillation drive unit (19) and the ones possessed by the high-frequency electric current output unit (25).

7. The ultrasonic surgical operation system according to claim 1, further comprising
a connection unit for connecting said high-frequency electric current output unit (25) to said ultrasonic oscillation drive unit for allowing them to communicate with each other, wherein
the connection unit enables an output instruction of said high-frequency electric current and said ultrasonic oscillation using said instruction of at least either of said first or second output instruction units.

## Patentansprüche

1. Chirurgisches Ultraschalloperationssystem, aufweisend:
ein Handstück (3) mit einer Sonde, an die eine Ultraschallschwingung und ein elektrischer Hochfrequenzstrom übermittelt werden;
eine Ultraschallschwingungsansteuerungseinheit (19), die in der Lage ist, ein Ultraschallschwingungssignal zu erzeugen und an das Handstück abzugeben, um die Sonde per Ultraschall zu oszillieren, und in der Lage ist, die Abgabemodi der Ultraschallschwingung zu ändern;
eine elektrische Hochfrequenzstromabgabeeinheit (25), die in der Lage ist, den elektrischen Hochfrequenzstrom zum Handstück abzugeben, und in der Lage ist, die Abgabemodi des elektrischen Hochfrequenzstroms zu ändern;
eine erste Abgabeanweisungseinheit (5) zum Abgeben einer Anweisung an die Ultraschallschwingungsansteuerungseinheit, um das Ultraschallschwingungssignal abzugeben;
eine zweite Abgabeanweisungseinheit (14) zum Abgeben einer Anweisung an die elektrische Hochfrequenzstromabgabeeinheit, um den elektrischen Hochfrequenzstrom abzugeben;
eine Umschaltanweisungseinheit (13) zum Abgeben einer Anweisung, dass die Abgabemodi und die Abgabewerte der Ultraschallschwingungsansteuerungseinheit (19) und/oder der elektrischen Hochfrequenzstromabgabeeinheit (25) umgeschaltet werden; und
eine Steuerungseinheit (16, 22), die dazu ausgebildet ist, die Abgabemodi und die Abgabewerte der Ultraschallschwingungsansteuerungseinheit (19) und/oder der elektrischen Hochfrequenzstromabgabeeinheit (25) auf Grundlage der Anweisung von der Umschaltanweisungseinheit (13) umzuschalten, um wahlweise entweder die Abgabemodi oder die Abgabewerte gemäß der Anweisung von der ersten Abgabeanweisungseinheit (5) oder der zweiten Abgabeanweisungseinheit (14) umzuschalten,
wobei die Steuerungseinheit (16, 22) ferner dazu ausgebildet ist, die Abgabemodi des elektrischen Hochfrequenzstroms (25) und/oder der Ultraschallschwingung (19) auf Grundlage der Anweisung von der Umschaltanweisungseinheit (13) umzuschalten, falls keine Abgabeanweisung entweder von der ersten Abgabeanweisungseinheit (5) oder der zweiten Abgabeanweisungseinheit (14) vorhanden ist, oder um die Abgabewerte des elektrischen Hochfrequenzstroms und/oder der Ultraschallschwingung auf Grundlage der Anweisung von der Umschaltanweisungseinheit (13) zu ändern, falls die Abgabeanweisung entweder von der ersten Abgabeanweisungseinheit (5) oder der zweiten Abgabeanweisungseinheit (14) vorhanden ist, und
die Umschaltanweisungseinheit (13) aus einem einzigen Schalter gebildet ist, der auf dem Handstück (3) vorgesehen ist.

2. Chirurgisches Ultraschalloperationssystem nach Anspruch 1, wobei
die erste Abgabeanweisungseinheit (5), die zweite Abgabeanweisungseinheit (14) und die Umschaltanweisungseinheit (13) die entsprechenden Anweisungssignale an die Ultraschallschwingungsansteuerungseinheit (19), die elektrische Hochfrequenzstromabgabeeinheit (25) und die Steuerungseinheit (16, 22) abgeben, um diesen Anweisungen zu erteilen;
wobei die Steuerungseinheit aufweist:
eine oder mehrere Anweisungssignalermittlungseinheiten zum Ermitteln des Anweisungssignals, das von der ersten Abgabeanweisungseinheit (5), der zweiten Abgabeanweisungseinheit (14) oder der Umschaltanweisungseinheit (13) abgegeben wird, und
eine oder mehrere Umschaltsteuerungseinheiten (16, 22) zum Umschalten der Abgabemodi des elektrischen Hochfrequenzstroms und/oder der Ultraschallschwingung auf Grundlage des Anweisungssignals, das durch die Anweisungssignalermittlungseinheit ermittelt wird, und zum Umschalten der Abgäbewerte des elektrischen Hochfrequenzstroms und/oder der Ultraschallschwingung im umgeschalteten Abgabemodus; und
wobei die Umschaltsteuerungseinheit (16, 22) dazu ausgebildet ist, die Abgabemodi auf Grundlage eines Anweisungssignals auszugeben, falls lediglich das Anweisungssignal von der Umschaltanweisungseinheit durch die Anweisungssignalermittlungseinheit (15, 21) ermittelt wird, oder um die Abgabewerte auf Grundlage eines Anweisungssignals umzuschalten, falls das Anweisungssignal entweder von der ersten Abgabeanweisungseinheit oder der zweiten Abgabeanweisungseinheit oder das Anweisungssignal von der Umschaltanweisungseinheit durch die Anweisungssignalermittlungseinheit ermittelt werden.

3. Chirurgisches Ultraschalloperationssystem nach Anspruch 1, wobei
die Umschaltanweisungseinheit ein Schalter (13) ist, der im Handstück (3) eingebaut ist.

4. Chirurgisches Ultraschalloperationssystem nach Anspruch 1, wobei
die erste Abgabeanweisungseinheit (5) ein Fußschalter oder ein Schalter (14) ist, der im Handstück (3) eingebaut oder daran beliebig abnehmbar angebracht ist.

5. Chirurgisches Ultraschalloperationssystem nach Anspruch 1, wobei
die zweite Abgabeanweisungseinheit (14) ein Fußschalter oder ein Schalter ist, der im Handstück (3) eingebaut ist oder daran beliebig abnehmbar angebracht ist.

6. Chirurgisches Ultraschalloperationssystem nach Anspruch 1, wobei
die durch die Ultraschallschwingungsansteuereinheit (19) verarbeiteten Abgabemodi einen festgelegten Ultraschallabgabemodus zum Abgeben einer Ultraschallschwingung mit einem festgelegten Abgabewert, einen Maximalultraschallabgabemodus zum Abgeben einer Ultraschallschwingung mit einem Abgabewert am oberen Grenzwert und/oder einen Null-Abgabemodus aufweist;
wobei die durch die elektrische Hochfrequenzstromabgabeeinheit (25) verarbeiteten Abgabemodi einen elektrischen Hochfrequenzstromabgabemodus zur Verwendung bei der Inzision, einen elektrischen Hochfrequenzstromabgabemodus zur Verwendung bei der Koagulation und/oder einen Null-Abgabemodus aufweisen und
wobei die zum Umschalten durch die Steuerungseinheit (16, 22) zulässigen Abgabemodi Abgabemodi sind, die durch Kombinationen der Abgabemodi, die die Ultraschallschwingungsansteuerungseinheit (19) aufweist, und diejenigen gebildet werden, die die elektrische Hochfrequenzstromabgabeeinheit (25) aufweist.

7. Chirurgisches Ultraschalloperationssystem nach Anspruch 1, ferner aufweisend
eine Verbindungseinheit zum Verbinden der elektrischen Hochfrequenzstromabgabeeinheit (25) mit der Ultraschallschwingungsansteuerungseinheit, um ihnen zu ermöglichen, miteinander zu kommunizieren, wobei
die Verbindungseinheit eine Abgabeanweisung des elektrischen Hochfrequenzstroms und der Ultraschallschwingung unter Verwendung der Anweisung von der ersten Abgabeanweisungseinheit und/oder der zweiten Abgabeeinheit ermöglicht.

## Revendications

1. Système d'intervention chirurgicale par ultrasons, comprenant :
une pièce à main (3) comportant une sonde à laquelle une oscillation ultrasonore et un courant électrique de haute fréquence sont transmis ;
une unité d'attaque par oscillation ultrasonore (19) apte à engendrer et à délivrer en sortie à la pièce à main un signal d'oscillation ultrasonore afin de faire osciller la sonde par ultrasons, et à changer de mode de sortie de l'oscillation ultrasonore ;
une unité de sortie de courant électrique de haute fréquence (25) apte à délivrer en sortie le courant électrique de haute fréquence à la pièce à main et à changer de mode de sortie du courant électrique de haute fréquence ;
une première unité d'instruction de sortie (5) destinée à donner à l'unité d'attaque par oscillation ultrasonore l'instruction d'émettre en sortie le signal d'oscillation ultrasonore ;
une seconde unité d'instruction de sortie (14) destinée à donner à l'unité de sortie de courant électrique de haute fréquence l'instruction de délivrer en sortie le courant électrique de haute fréquence ;
une unité d'instruction de commutation (13) destinée à donner l'instruction de commuter les modes de sortie et les valeurs de sortie d'au moins l'une parmi l'unité d'attaque par oscillation ultrasonore (19) et l'unité de sortie de courant électrique de haute fréquence (25) ; et
une unité de commande (16, 22) qui est conçue pour commuter les modes de sortie et les valeurs de sortie d'au moins l'une parmi l'unité d'attaque par oscillation ultrasonore (19) et l'unité de sortie de courant électrique de haute fréquence (25) sur la base de l'instruction provenant de l'unité d'instruction de commutation (13), pour commuter de manière sélective soit les modes de sortie soit les valeurs de sortie en fonction de l'instruction donnée par la première (5) ou la seconde unité d'instruction de sortie (14),
dans lequel ladite unité de commande (16, 22) est conçue en outre pour commuter lesdits modes de sortie dudit courant électrique de haute fréquence (25) et/ou de ladite oscillation ultrasonore (19) sur la base de ladite instruction provenant de ladite unité d'instruction de commutation (13) s'il n'existe pas d'instruction de sortie provenant de l'une ou l'autre desdites première (5) et seconde (14) unités d'instruction de sortie, ou pour changer des valeurs de sortie d'au moins l'un ou l'autre du courant électrique de haute fréquence et de l'oscillation ultrasonore, sur la base de l'instruction provenant de l'unité d'instruction de commutation (13) s'il existe l'instruction de sortie provenant de l'une ou l'autre desdites première (5) et seconde (14) unités d'instructions de sortie, et
ladite unité d'instruction de commutation (13) est constituée d'un commutateur unique équipant la pièce à main (3).

2. Système d'intervention chirurgicale par ultrasons selon la revendication 1,
dans lequel :
ladite première unité d'instruction de sortie (5), ladite seconde unité d'instruction de sortie (14) et ladite unité d'instruction de commutation (13) émettent en sortie des signaux d'instructions respectifs vers ladite unité d'attaque par oscillation ultrasonore (19), ladite unité de sortie de courant électrique de haute fréquence (25) et ladite unité de commande (16, 22), pour ladite instruction leur correspondant ;
l'unité de commande comprend :
une ou plusieurs unités de détection de signaux d'instructions destinées à détecter le signal d'instruction émis en sortie de la première unité d'instruction de sortie (5), la seconde unité d'instruction de sortie (14) ou l'unité d'instruction de commutation (13), et
une ou plusieurs unités de commande de commutation (16, 22) destinées à commuter lesdits modes de sortie d'au moins l'un ou l'autre parmi ledit courant électrique de haute fréquence et ladite oscillation ultrasonore, sur la base du signal d'instruction détecté par l'unité de détection de signaux d'instructions (15, 21), et à commuter lesdites valeurs de sortie d'au moins l'un ou l'autre parmi le courant électrique de haute fréquence et l'oscillation ultrasonore dans le mode de sortie commuté ; et
dans lequel l'unité de commande de commutation (16, 22) est conçue pour commuter les modes de sortie sur la base d'un signal d'instruction si seul le signal d'instruction provenant de l'unité d'instruction de commutation est détecté par l'unité de détection de signaux d'instructions (15, 21), ou pour commuter les valeurs de sortie sur la base d'un signal d'instruction si le signal d'instruction provenant de l'une ou l'autre des première et seconde unités d'instruction de sortie et le signal d'instruction provenant de l'unité d'instruction de commutation sont détectés par l'unité de détection de signaux d'instructions.

3. Système d'intervention chirurgicale par ultrasons selon la revendication 1, dans lequel :
ladite unité d'instruction de commutation est un commutateur (13) incorporé à ladite pièce à main (3).

4. Système d'intervention chirurgicale par ultrasons selon la revendication 1, dans lequel :
ladite première unité d'instruction de sortie (5) est un commutateur à pédale ou un commutateur (14) incorporé ou fixé de manière librement détachable à ladite pièce à main (3).

5. Système d'intervention chirurgicale par ultrasons selon la revendication 1, dans lequel :
ladite seconde unité d'instruction de sortie (14) est un commutateur à pédale ou un commutateur incorporé ou fixé de manière librement détachable à ladite pièce à main (3).

6. Système d'intervention chirurgicale par ultrasons selon la revendication 1, dans lequel :
lesdits modes de sortie que possède ladite unité d'attaque par oscillation ultrasonore (19) comprennent au moins l'un parmi un mode de sortie ultrasonore fixe pour émettre en sortie une oscillation ultrasonore d'une valeur de sortie fixe, un mode de sortie ultrasonore maximum pour émettre en sortie une oscillation ultrasonore d'une valeur de sortie de limite supérieure, ou un mode de sortie nulle ;
lesdits modes de sortie que possède ladite unité de sortie de courant électrique de haute fréquence (25), comprennent au moins l'un parmi un mode de sortie de courant électrique de haute fréquence pour une utilisation en incision, un mode de sortie de courant électrique de haute fréquence pour une utilisation en coagulation ou un mode de sortie nulle, et
lesdits modes autorisés pour la commutation par ladite unité de commande (16, 22) sont des modes de sortie constitués par des combinaisons des modes de sortie que possède l'unité d'attaque par oscillation ultrasonore (19) et ceux que possède l'unité de sortie de courant électrique de haute fréquence (25).

7. Système d'intervention chirurgicale par ultrasons selon la revendication 1, comprenant en outre :
une unité de raccordement destinée à raccorder ladite unité de sortie de courant électrique de haute fréquence (25) à ladite unité d'attaque par oscillation ultrasonore pour leur permettre de communiquer l'une avec l'autre, dans lequel :
l'unité de raccordement valide une instruction de sortie dudit courant électrique de haute fréquence et de ladite oscillation ultrasonore à l'aide de ladite instruction d'au moins l'une ou l'autre desdites première et seconde unités d'instructions de sortie.
